# EUROPEAN PATENT SPECIFICATION

(11) **EP 3 311 164 B1**
(45) Date of publication and mention of the grant of the patent: **20.04.2022**
(21) Application number: 16812578.9
(22) Date of filing: 17.06.2016
(51) Int. Cl.: G01N 33/573, G01N 33/53

(54) **METHODS AND COMPOSITIONS FOR DIAGNOSIS AND PROGNOSIS OF APPENDICITIS AND DIFFERENTIATION OF CAUSES OF ABDOMINAL PAIN**
VERFAHREN UND ZUSAMMENSETZUNGEN ZUR DIAGNOSE UND PROGNOSE VON APPENDIZITIS UND DIFFERENZIERUNG VON URSACHEN FÜR BAUCHSCHMERZEN
MÉTHODES ET COMPOSITIONS POUR LE DIAGNOSTIC ET LE PRONOSTIC DE L'APPENDICITE ET LA DIFFÉRENCIATION DES CAUSES DE LA DOULEUR ABDOMINALE

(30) Priority: 17.06.2015 US 201562181055 P
(43) Date of publication of application: 25.04.2018
(73) Proprietor: Astute Medical, Inc., San Diego, CA 92121 (US); Children's Medical Center Corporation, Boston, MA 02115 (US)
(72) Inventor: McPHERSON, Paul, Encinitas, CA 92024 (US); KAMPF, James Patrick, San Diego, CA 92130 (US); KWAN, Thomas, San Diego, CA 92122 (US); BACHUR, Richard, North Reading, MA 01864 (US); STEEN, Hanno, Cambridge, MA 02139 (US)
(74) Representative: Wilding, James Roger
(86) International application number: PCT/US2016/038225
(87) International publication number: WO 2016/205740

(56) References cited:
- WO-A1-2010/078411
- WO-A1-2013/163345
- WO-A1-2015/042465
- US-A1- 2015 031 049
- US-A1- 2015 031 049
- Magdalena Schröckenfuchs: "Hepcidin als diagnostischer marker der Appendizitis im Kindersalter", Diplomarbeit, 5 March 2015 (2015-03-05), XP055511809, Retrieved from the Internet: URL:https://online.medunigraz.at/mug_onlin e/webnav.ini, AND in the search field : "Thesis, Final Papers" ,author : "Schröckenfuchs", year : 2015, [retrieved on 2018-10-02]
- ECE YAPAKÇI ET AL: "Serum pro-hepcidin levels in term and preterm newborns with sepsis", PEDIATRICS INTERNATIONAL, vol. 51, no. 2, 1 April 2009 (2009-04-01), pages 289-292, XP055512284, XX ISSN: 1328-8067, DOI: 10.1111/j.1442-200X.2008.02688.x
- KENTSIS ALEX ET AL: "Urine proteomics for profiling of human disease using high accuracy mass spectrometry", PROTEOMICS - CLINICAL APPLICAT, WILEY, DE, vol. 3, no. 9, 1 September 2009 (2009-09-01), pages 1052-1061, XP009162639, ISSN: 1862-8346, DOI: 10.1002/PRCA.200900008
- XIANG ET AL.: 'Detection of D-3-phosphoglycerate dehydrogenase autoantibodies in patients with autoimmune hepatitis: clinical significance evaluation' HEPATOLOGY RESEARCH vol. 41, no. 9, 2011, pages 867 - 876, XP055338978

## Description

The present invention claims priority from U.S. Provisional Patent Application 62/181,055 filed June 17, 2015.

### BACKGROUND OF THE INVENTION

The following discussion of the background of the invention is merely provided to aid the reader in understanding the invention and is not admitted to describe or constitute prior art to the present invention.

Acute appendicitis is an inflammatory condition which typically results from a primary obstruction of the appendix lumen. Once obstructed, the appendix subsequently swells, increasing pressures within the lumen and the walls of the appendix, resulting in thrombosis and occlusion of the small vessels, and stasis of lymphatic flow. The causative agents of appendicitis include foreign bodies, trauma, intestinal worms, lymphadenitis, and, most commonly, calcified fecal deposits known as appendicoliths or fecaliths. Diagnosis is based on patient history, symptoms and physical examination. Typical appendicitis usually includes abdominal pain beginning in the region of the umbilicus for several hours, associated with anorexia, nausea or vomiting. The pain typically settles into the right lower quadrant.

A commonly used acronym for diagnosis is PALF: pain, anorexia, leukocytosis, and fever. Atypical histories lack this typical progression and may include pain in the right lower quadrant as an initial symptom. Atypical histories often require imaging with ultrasound and/or CT scanning. Blood tests for appendicitis have limited diagnostic value. These tests tend to be relatively simple. An abnormal rise in the number of white blood cells in the blood is a crude indicator of infection or inflammation going on in the body. Such a rise is not specific to appendicitis alone. If it is abnormally elevated, with a good history and examination findings pointing towards appendicitis, the likelihood of having the disease is higher. Imaging tests such as CT, while useful, expose the recipient to diagnostic levels of radiation.

In terms of biomarkers, C-reactive protein (CRP), an acute-phase response protein produced by the liver in response to inflammatory processes, has been used by clinicians. Likewise, other general inflammatory markers such as procalcitonin, Interleukin-6 (IL-6), Interleukin-8 (IL-8), high mobility group box-1 protein (HMGB 1), S 100A8/A9, etc., have been studied by clinicians. Like the number of white blood cells, however, these are not specific biomarkers of appendicitis, and so exhibit poor specificity in use. Leucine-rich alpha-2-glycoprotein (LRG) was recently suggested to be a more specific indicator of appendicitis in a pediatric population. Kentsis et al., Ann. Emerg. Med. 55: 62-70.e4. Epub 2009 Jun 25; Kharbanda et al., Academic Emerg. Med. 19: 56-62, 2012.

Schröckenfuchs, Diplomarbeit (2015), Medical University of Graz (URL: https://online.medunigraz.at/ mug_online/webnav.ini) mentions hepcidin as a diagnostic marker of childhood appendicitis.

There remains a need in the art for a rapid, objective, clinically accurate, available diagnostic tool for aiding in the diagnosis and care of appendicitis.

### BRIEF SUMMARY OF THE INVENTION

The present invention provides methods and uses as defined in the attached claims.

The present invention relates to the use of Pro-Hepcidin as a diagnostic marker for appendicitis. The claimed methods and uses can meet the need in the art for rapid, sensitive and specific diagnostic assay to be used in the identification of appendicitis.

In one aspect, the invention provides methods for diagnosing appendicitis in a subject. The subject may be being evaluated for abdominal pain. The methods comprise performing an assay configured to detect Pro-Hepcidin on a body fluid sample obtained from the subject to provide an assay result; and correlating the assay result to the occurrence or nonoccurrence of appendicitis in the subject by comparing the assay result to a threshold selected in a population study, wherein the threshold separates the population into a first subpopulation above the threshold which is at an increased predisposition for the occurrence of appendicitis, and a second subpopulation below the threshold which is at an increased predisposition for the nonoccurrence of appendicitis relative to the first subpopulation.

Pro-Hepcidin may be used, individually or in panels comprising a plurality of appendicitis biomarkers. The presence or amount of Pro-Hepcidin in a sample obtained from the subject can be used to rule in or rule out appendicitis.

In another aspect, the present invention relates to methods for evaluating Pro-Hepcidin levels in a body fluid sample, comprising: performing an analyte binding assay configured to detect Pro-Hepcidin in a body fluid sample obtained from a subject selected for evaluation based on a determination that the subject is experiencing symptoms indicative of possible acute appendicitis, or the subject has been diagnosed with acute appendicitis, wherein the body fluid sample is introduced into an assay instrument which (i) contacts the body fluid sample with one or more binding reagents, wherein Pro-Hepcidin binds to the one or more binding reagents in an amount related to its concentration in the body fluid sample, (ii) generates an assay result indicative of binding of Pro-Hepcidin to the one or more binding reagents, and (iii) displays the assay result as a quantitative result in a human-readable form. In one embodiment, the assay result indicates that the body fluid sample has an elevated level of Pro-Hepcidin as compared to a predetermined threshold level.

The predetermined threshold may be selected to be indicative of one or more of the following: diagnosis of acute appendicitis; indication of a prognosis resulting from acute appendicitis. For convenience, patients being evaluated in this manner are referred to herein as "appendicitis patients," whether or not the appendicitis diagnosis has been confirmed at the time of the evaluation.

The methods for evaluating Pro-Hepcidin levels can be used for risk stratification of the patient; that is, assigning a likelihood of one or more future changes in health status to the patient, wherein the assay result correlates to one or more such future changes. A level or a change in level of the appendicitis biomarker , which in turn is(are) associated with an increased probability of morbidity or failure of medical therapy are referred to as being "associated with an increased predisposition to an adverse outcome" in a patient.

In such risk stratification , the likelihood or risk may be that an event of interest is more or less likely to occur within 180 days of the time at which the body fluid sample is obtained from the appendicitis patient , or the likelihood or risk may relate to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the appendicitis patient is equivalent to diagnosis of a current condition.

For a positive going marker, an increased likelihood of the occurrence of a diagnosis is assigned to the patient when the measured concentration is above the threshold (relative to the likelihood assigned when the measured concentration is below the threshold); alternatively, when the measured concentration is below the threshold, an increased likelihood of the nonoccurrence of a diagnosis may be assigned to the patient (relative to the likelihood assigned when the measured concentration is above the threshold). For a negative going marker, an increased likelihood of the occurrence of a diagnosis is assigned to the patient when the measured concentration is below the threshold (relative to the likelihood assigned when the measured concentration is above the threshold); alternatively, when the measured concentration is above the threshold, an increased likelihood of the nonoccurrence of a diagnosis may be assigned to the patient (relative to the likelihood assigned when the measured concentration is below the threshold). In the methods and uses of the invention, Pro-Hepcidin is used as a marker.

A threshold level of Pro-Hepcidin can be established, and the level of the indicator in a patient sample can simply be compared to the threshold level. A variety of methods may be used by the skilled artisan to arrive at a desired threshold value.

For example, the threshold value may be determined from a population of patients not having acute appendicitis by selecting a concentration representing the 75^{th}, 85^{th}, 90^{th}, 95^{th}, or 99^{th} percentile of the appendicitis biomarker measured in such "normal" patients. Alternatively, the threshold value may be determined from a "diseased" population of patients by selecting a concentration representing the 75^{th}, 85^{th}, 90^{th}, 95^{th}, or 99^{th} percentile of the biomarker measured in patients suffering from acute appendicitis.

Alternatively, the threshold value may be determined from a "diseased" population of appendicitis patients having a predisposition for an outcome such as death, worsening disease, *etc.*), by selecting a concentration representing the 75^{th}, 85^{th}, 90^{th}, 95^{th}, or 99^{th} percentile of the biomarker measured in patients suffering from acute appendicitis and who later suffered from the outcome of interest.

In another alternative, the threshold value may be determined from a prior measurement of a biomarker or biomarkers in the same patient; that is, a temporal change in the level of a biomarker or biomarkers in the same patient may be used to assign a diagnosis or a prognosis to the patient. For example, a diagnostic indicator may be determined at an initial time, and again at a second time. In such embodiments, an increase in the marker from the initial time to the second time may be diagnostic of appendicitis or a given prognosis. In the methods and uses of the invention, the Pro-Hepcidin biomarker is measured.

The foregoing discussion is not meant to imply, however, that the appendicitis biomarker used in the present invention must be compared to a corresponding individual threshold . Methods for combining assay results can comprise the use of multivariate logistical regression, loglinear modeling, neural network analysis, n-of-m analysis, decision tree analysis, calculating ratios of markers, *etc.* This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which has a particular disease (or which is predisposed to some outcome), and a "second" subpopulation which does not have the disease (or is not so predisposed) can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of Pro-Hepcidin, or a composite of appendicitis biomarkers including Pro-Hepcidin, may be treated as continuous variables. For example, any particular concentration can be converted into a corresponding probability of existing disease, of a future outcome for the appendicitis patient, or mortality, of a SIRS classification, etc. In yet another alternative, a threshold that can provide an acceptable level of specificity and sensitivity in separating a population of appendicitis patients into "bins" such as a "first" subpopulation and a "second" subpopulation. A threshold value is selected to separate this first and second population by one or more of the following measures of test accuracy:
an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;
a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;
a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;
at least about 75% sensitivity, combined with at least about 75% specificity;
a positive likelihood ratio (calculated as sensitivity/(1-specificity)) of greater than 1, at least about 2, more preferably at least about 3, still more preferably at least about 5, and most preferably at least about 10; or
a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/-5% of a given measurement.

Multiple thresholds may also be used to assess a patient. For example, a "first" subpopulation identified by an existing disease, predisposition to a future outcome for the appendicitis patient, predisposition to mortality, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to appendicitis patients based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in disease status in comparison to someone in the first tertile. The third tertile is also assigned an odds ratio that is relative to that first tertile.

In certain embodiments, the assay method is an immunoassay. Antibodies for use in such assays will specifically bind a full length appendicitis biomarker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma.

The foregoing method steps should not be interpreted to mean that the appendicitis biomarker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the appendicitis patient selected from the group consisting of demographic information (e.g., weight, sex, age, race), clinical variables (e.g., blood pressure, temperature, respiration rate), risk scores (Alvarado score, Pediatric Appendicitis Score, etc.). This list is not meant to be limiting.

When more than one marker is measured, the individual markers may be measured in samples obtained at the same time, or may be determined from samples obtained at different (e.g., an earlier or later) times. The individual markers may also be measured on the same or different body fluid samples. For example, one appendicitis biomarker may be measured in a serum or plasma sample and another appendicitis biomarker may be measured in a urine sample. In addition, assignment of a likelihood may combine an individual biomarker assay result with temporal changes in one or more additional variables. In the methods and uses of the invention, the Pro-Hepcidin marker is measured.

In various related aspects, the present invention employs devices for performing the methods described herein.

In certain embodiments, reagents for performing such assays are provided in an assay device. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, *etc.)* as well as molecules that may be indirectly detected by production of a detectable reaction product (e.g., enzymes such as horseradish peroxidase, alkaline phosphatase, *etc.)* or through the use of a specific binding molecule which itself may be detectable (*e.g.,* a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, *etc.* In certain of these methods, the solid phase antibody is coupled to a transducer (*e.g*., a diffraction grating, electrochemical sensor, etc) for generation of a signal, while in others, a signal is generated by a transducer that is spatially separate from the solid phase antibody (*e.g*., a fluorometer that employs an excitation light source and an optical detector). This list is not meant to be limiting. Antibody-based biosensors may also be employed to determine the presence or amount of analytes that optionally eliminate the need for a labeled molecule.

### DETAILED DESCRIPTION

The technical information set out below may in some respects go beyond the scope of the invention, which is defined exclusively by the attached claims. The additional technical information is provided to place the actual invention in a broader technical context and to illustrate possible related technical developments. The disclosure relates to methods and compositions for diagnosis, differential diagnosis, risk stratification, monitoring, classifying and determination of treatment regimens in patients diagnosed with, or at risk of, appendicitis. A measured concentration of one or more biomarkers selected from the group consisting of D-3-phosphoglycerate dehydrogenase, Vacuolar protein sorting-associated protein 4B, Keratin (type II cytoskeletal 75), Ig kappa chain V-III region B6, Ig lambda chain V-I region NIG-64, Cystatin-B, Alpha-1B-glycoprotein, Ig kappa chain V-I region BAN, Keratin (type I cytoskeletal 18), Keratin (type II cytoskeletal 8), Ig lambda chain V-I region BL2, Neurofilament medium polypeptide, Protein disulfide-isomerase, Calpain-1 catalytic subunit, Vimentin, Keratin (type II cytoskeletal 7), Complement Cls subcomponent, Complement component C7, Annexin A3, Arylsulfatase A, 15-hydroxyprostaglandin dehydrogenase [NAD(+)], Desmin, Ganglioside GM2 activator, Myosin-9, Myosin-11, Transgelin-2, Transaldolase, Interferon alpha/beta receptor 2, decarboxylating 6-phosphogluconate dehydrogenase, F-actin-capping protein subunit alpha-1, ATP-citrate synthase, Dipeptidyl peptidase 1, Alpha-N-acetylglucosaminidase, Eukaryotic translation initiation factor 6, Actin-related protein 2, Pro-Hepcidin, Heterogeneous nuclear ribonucleoprotein U, Adenylyl cyclase-associated protein 1, Alpha-1,6-mannosylglycoprotein 6-beta-N-acetylglucosaminyltransferase A, Hyaluronidase-1, Alpha-internexin, Myomegalin, Peptidase inhibitor 16, CDK5 regulatory subunit-associated protein 2, Protease-associated domain-containing protein 1, Sialate O-acetylesterase, Transgelin-3, Junctional adhesion molecule A, Carboxypeptidase Q, and Adseverin or one or more markers related thereto, can be correlated to the status of the patient. As described herein, measurement of one or more biomarkers may be used, individually or in panels comprising a plurality of biomarkers, in methods and compositions for the diagnosis, prognosis, or differentiation of abdominal pain in order to rule in or out appendicitis and/or a particular outcome. Such markers can be used in diagnosing and treating a subject and/or to monitor the course of a treatment regimen; for screening subjects for the occurrence or risk of a particular disease; and for screening compounds and pharmaceutical compositions that might provide a benefit in treating or preventing such conditions.

For purposes of this document, the following definitions apply:
The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology. An "appendicitis patient" is a patient exhibiting symptoms consistent with appendicitis and being evaluated for its presence, absence, or outcome

Conditions within the differential diagnosis include gallbladder attack, kidney infection, pneumonia, rheumatic fever, diabetic ketoacidosis, ectopic pregnancy, twisted ovarian cyst, hemorrhaging ovarian follicle, urinary tract infection, ulcerative colitis, pancreatitis, intestinal obstruction, pelvic inflammatory disease, diverticulitis, carcinoma of the colon, and aortic aneurysm. The biomarkers described herein can distinguish appendicitis from one or more of these mimicking conditions.

Preferably, an analyte such as an appendicitis biomarker is measured in a sample. Such a sample may be obtained from a patient, such as an appendicitis patient. Preferred samples are body fluid samples.

The term "body fluid sample" as used herein refers to a sample of bodily fluid obtained for the purpose of diagnosis, prognosis, classification or evaluation of an appendicitis patient of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for Pro-Hepcidin, optionally together with other clinical characteristics, to arrive at a diagnosis (that is, the occurrence or nonoccurrence) of a disease or condition. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the appendicitis patient relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity or mortality is referred to as being "indicative of an increased likelihood" of an adverse outcome in a patient.

As used herein, the term "relating a signal to the presence or amount" of an analyte reflects the following understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the appendicitis biomarkers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, *etc.*

In this regard, the skilled artisan will understand that the signals obtained from an immunoassay are a direct result of complexes formed between one or more antibodies and the target biomolecule (*i.e*., the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc.)* and nucleic acid measurements (mRNA quatitation). This list is not meant to be limiting. With regard to biomarkers which exist in one form as type-I, type-II, or GPI-anchored membrane proteins, such membrane proteins typically comprise a substantial extracellular domain, some or all of which can be detected as soluble forms present in aqueous samples such as blood, serum, plasma, urine, etc., either as cleavage products or as splice variants which delete an effective membrane spanning domain. Preferred assays detect soluble forms of these biomarkers.

The term "positive going" marker as that term is used herein refer to a marker that is determined to be elevated in patients suffering from a disease or condition, relative to those not suffering from that disease or condition. The term "negative going" marker as that term is used herein refer to a marker that is determined to be reduced in patients suffering from a disease or condition, relative to patients not suffering from that disease or condition.

### Appendicitis biomarkers

The following table provides a list of the biomarkers together with the Swiss-Prot entry number for the human precursor. As noted above, these biomarkers are referred to for convenience herein as "appendicitis biomarkers."

| **Swiss-Prot ID** | **Abbreviation** | **Preferred Name** |
|---|---|---|
| O43175 | SERA | D-3-phosphoglycerate dehydrogenase |
| O75351 | VPS4B | Vacuolar protein sorting-associated protein 4B |
| O95678 | K2C75 | Keratin, type II cytoskeletal 75 |
| P01619 | KV301 | Ig kappa chain V-III region B6 |
| P01702 | LV 104 | Ig lambda chain V-I region NIG-64 |
| P04080 | CYTB | Cystatin-B |
| P04217 | A1BG | Alpha-1B-glycoprotein |
| P04430 | KV122 | Ig kappa chain V-I region BAN |
| P05783 | K1C18 | Keratin, type I cytoskeletal 18 |
| P05787 | K2C8 | Keratin, type II cytoskeletal 8 |
| P06316 | LV107 | Ig lambda chain V-I region BL2 |
| P07197 | NFM | Neurofilament medium polypeptide |
| P07237 | PDIA1 | Protein disulfide-isomerase |
| P07384 | CAN1 | Calpain-1 catalytic subunit |
| P08670 | VIME | Vimentin |
| P08729 | K2C7 | Keratin, type II cytoskeletal 7 |
| P09871 | C1S | Complement C1s subcomponent |
| P10643 | CO7 | Complement component C7 |
| P12429 | ANXA3 | Annexin A3 |
| P15289 | ARSA | Arylsulfatase A |
| P15428 | PGDH | 15-hydroxyprostaglandin dehydrogenase [NAD(+)] |
| P17661 | DESM | Desmin |
| P17900 | SAP3 | Ganglioside GM2 activator |
| P35579 | MYH9 | Myosin-9 |
| P35749 | MYH11 | Myosin-11 |
| P37802 | TAGL2 | Transgelin-2 |
| P37837 | TALDO | Transaldolase |
| P48551 | INAR2 | Interferon alpha/beta receptor 2 |
| P52209 | 6PGD | 6-phosphogluconate dehydrogenase, decarboxylating |
| P52907 | CAZA1 | F-actin-capping protein subunit alpha-1 |
| P53396 | ACLY | ATP-citrate synthase |
| P53634 | CATC | Dipeptidyl peptidase 1 |
| P54802 | ANAG | Alpha-N-acetylglucosaminidase |
| P56537 | IF6 | Eukaryotic translation initiation factor 6 |
| P61160 | ARP2 | Actin-related protein 2 |
| P81172 | HEPC | Pro-Hepcidin |
| Q00839 | HNRPU | Heterogeneous nuclear ribonucleoprotein U |
| Q01518 | CAP1 | Adenylyl cyclase-associated protein 1 |
| Q09328 | MGT5A | Alpha-1,6-mannosylglycoprotein 6-beta-N-acetylglucosaminyltransferase A |
| Q12794 | HYAL1 | Hyaluronidase-1 |
| Q16352 | AINX | Alpha-intemexin |
| Q5VU43 | MYOME | Myomegalin |
| Q6UXB8 | PI16 | Peptidase inhibitor 16 |
| Q96SN8 | CK5P2 | CDK5 regulatory subunit-associated protein 2 |
| Q9BSG0 | PADC1 | Protease-associated domain-containing protein 1 |
| Q9HAT2 | SIAE | Sialate O-acetylesterase |
| Q9UI15 | TAGL3 | Transgelin-3 |
| Q9Y624 | JAM1 | Junctional adhesion molecule A |
| Q9Y646 | CBPQ | Carboxypeptidase Q |
| Q9Y6U3 | ADSV | Adseverin |

### Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377.

One skilled in the art also recognizes that robotic instrumentation including but not limited to Beckman ACCESS^{®}, Abbott AXSYM^{®}, Roche ELECSYS^{®}, Dade Behring STRATUS^{®} systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (*e.g.*, fluorescent moieties, electrochemical labels, metal chelates, *etc.)* as well as molecules that may be indirectly detected by production of a detectable reaction product (e.g., enzymes such as horseradish peroxidase, alkaline phosphatase, *etc.)* or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homofunctional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

Described herein are kits for the analysis of the described appendicitis biomarkers. The kit comprises reagents for the analysis of at least one test sample which comprise at least one antibody that binds an appendicitis biomarker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies bind an appendicitis biomarker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

### Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. See, e.g. Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to an appendicitis biomarker . The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, preferred antibodies bind with affinities of at least about 10⁷ M⁻¹, and preferably between about 10⁸ M⁻¹ to about 10⁹ M⁻¹, about 10⁹ M⁻¹ to about 10¹⁰ M⁻¹, or about 10¹⁰ M⁻¹ to about 10¹² M⁻¹.

Affinity is calculated as Kd = k_{off}/kₒₙ (k_{off} is the dissociation rate constant, Kₒₙ is the association rate constant and K_{d} is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098 .

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides can confer improved characteristics on the ligand, such as improved in vivo stability or improved delivery characteristics. Examples of such modifications include chemical substitutions at the ribose and/or phosphate and/or base positions, and may include amino acid side chain functionalities.

### Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection therory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a patient belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the appendicitis biomarker assay result(s) of the present invention. Other clinical indicia which may be combined with the appendicitis biomarker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, or renal insufficiency), risk scores (APACHE score, PREDICT score, TIMI Risk Score for UA/NSTEMI, Framingham Risk Score), etc.

Combining assay results/clinical indicia in this manner can comprise the use of multivariate logistical regression, loglinear modeling, neural network analysis, n-of-m analysis, decision tree analysis, etc. This list is not meant to be limiting.

### Selecting a Treatment Regimen

Once a diagnosis is obtained, the clinician can readily select a treatment regimen that is compatible with the diagnosis. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers described herein may be used to monitor a course of treatment. For example, improved or worsened prognostic state may indicate that a particular treatment is or is not efficacious.

If appendicitis is diagnosed, surgery to remove the appendix is indicated to avoid its rupture. If the appendix has formed an abscess, two procedures may be performed; one to do a CT-guided drainage of the abscess, and a second one to remove the appendix eight to 12 weeks later. This delayed surgery is called an interval appendectomy. Antibiotics are given before an appendectomy to fight possible peritonitis, or infection of the abdominal cavity's lining. Appendectomy can be performed as open surgery using one abdominal incision about 2 to 4 inches (5 to 10 centimeters) long (laparotomy). Or the surgery can be done through a few small abdominal incisions (laparoscopic surgery).

### Example 1. Use of urine markers to diagnose acute pediatric appendicitis

Patients younger than 18 years old suspected of acute appendicitis by physicians in a hospital emergency department were enrolled in the study. Urine samples were collected from each patient, and the concentrations of proteins in a set of 89 urine samples were measured by mass spectroscopy. Of the 89 patients, 27 were classified as either having acute appendicitis as determined by a physician's gross and histologic examination and confirmed by a clinical pathologist's review of appendectomy specimens or not having acute appendicitis. (See Kentsis et al., Annals of Emergency Medicine 55(1), 62-70, 2010 for a detailed description of study.)

The raw intensity data from the mass spectrometer were processed by three different protein quantification methods: label-free quantification (LFQ), intensity-based absolute quantification (iBAQ), and data-independent acquisition (DIA). (See Cox et al., Molecular & Cellular Proteomics 13(9), 2513-2526, 2014, Schwanhäusser et al., Nature 473, 337-342, 2011, and Gillet et al., Molecular & Cellular Proteomics 11(6), 0111-016717, 2012, respectively.) The processed data were then analyzed by the receiver-operator characteristic (ROC) analysis to assess the ability of the proteins to predict acute pediatric appendicitis. In addition, ROC analysis was also performed on normalized intensity data where the intensity signal from each protein in a sample was first normalized by the intensity of albumin in the same sample.

**Table 1: Performance of various proteins by ROC analysis. For each protein, the area under the ROC curve (AUC) for each of the 6 datasets (LFQ, LFQ normalized, iBAQ, iBAQ normalized, DIA, DIA normalized) is categorized into the following manner: "A" denotes AUC greater or equal to 0.8, "B" denotes AUC greater than or equal to 0.75 but less than or 0.8, "C" denotes AUC greater than 0.5 but less than 0.75, "D" denotes AUC greater than 0.25 but less than or equal to 0.5, "E" denotes AUC greater than 0.2 but less than or equal to 0.25, and "F" denotes AUC less than or equal to 0.2. "ND" denotes no available intensity data for the protein in that dataset. Multiple proteins listed in a single row indicates that the mass spectrometry could not distinguish the proteins from one another.**

| **SwissProtID(Abbrevation)** | **LFQ** | **LFQ normalized** | **iBAQ** | **iBAQ normalized** | **DIA** | **DIA normalized** |
|---|---|---|---|---|---|---|
| Q9BSG0(PADC1) | C | D | C | C | C | B |
| P81172(HEPC) | B | C | C | B | C | C |
| P53634(CATC) | B | C | C | C | C | C |
| P15289(ARSA) | B | C | C | C | C | C |
| P53396(ACLY) | D | D | D | E | D | D |
| P52209(6PGD) | E | E | D | D | D | D |
| Q01518(CAP1) | D | D | E | E | D | D |
| P61160(ARP2) | D | D | E | E | D | D |
| P07237(PDIA1) | D | D | E | D | D | D |
| P37837(TALDO) | D | D | E | E | D | D |
| P05787(K2C8);O95678(K2C75) | ND | ND | ND | ND | D | E |
| P04080(CYTB) | D | D | D | D | E | D |
| Q00839(HNRPU) | D | D | D | D | E | D |
| O43175(SERA) | E | E | E | E | E | D |
| P07384(CAN1) | D | D | D | D | E | D |
| P12429(ANXA3) | D | D | D | D | E | D |
| P52907(CAZA1) | D | D | D | D | E | E |
| P08729(K2C7) | ND | ND | ND | ND | E | E |
| P15428(PGDH) | D | D | D | D | E | D |
| P37802(TAGL2);Q9UI15(TAGL3) | D | D | E | D | ND | ND |
| P05783(K1C18) | E | E | E | E | ND | ND |
| P35579(MYH9);P35749(MYH11) | F | E | D | D | ND | ND |

### Example 2. Use of urine markers to diagnose acute pediatric appendicitis

Patients 2-21 years of age presenting to the emergency department (ED) with signs and symptoms consistent with possible acute appendicitis are included in the study. Urine is collected from each patient at the time of the ED visit. Patients are classified as either having histologically proven appendicitis or not having histologically proven appendicitis.

The raw intensity data from the mass spectrometer were processed by two different protein quantification methods: label-free quantification (LFQ) and intensity-based absolute quantification (iBAQ), and two different sample preparation methods: MStern blotting and filter-aided sample preparation (FASP). (See Wisniewski et al., Nature Methods 6.5, 359-362, 2009.) The processed data were then analyzed by the ROC analysis to assess the ability of the proteins to predict acute pediatric appendicitis. In addition, ROC analysis was also performed on normalized intensity data where the intensity signal from each protein in a sample was first normalized by the intensity of albumin in the same sample.

Table 2: Performance of various proteins by ROC analysis. For each protein, the area under the ROC curve (AUC) for each of the 8 datasets (LFQ MStern, LFQ MStern normalized, iBAQ MStern, iBAQ MStern normalized, LFQ FASP, LFQ FASP normalized, iBAQ FASP, iBAQ FASP normalized) is categorized into the following manner: "A" denotes AUC greater or equal to 0.8, "B" denotes AUC greater than or equal to 0.75 but less than or 0.8, "C" denotes AUC greater than 0.5 but less than 0.75, "D" denotes AUC greater than 0.25 but less than or equal to 0.5, "E" denotes AUC greater than 0.2 but less than or equal to 0.25, and "F" denotes AUC less than or equal to 0.2. "ND" denotes no available intensity data for the protein in that dataset. Multiple proteins listed in a single row indicates that the mass spectrometry could not distinguish the proteins from one another.

| **SwissProtID (Abbreviation)** | **LFQ MSter n** | **LFQ MSter n norm alized** | **iBAQ MSter n** | **iBAQ MSter n norm alized** | **LF Q FAS P** | **LFQ FASP norm alized** | **iBA Q FAS P** | **iBAQ FASP norma lized** |
|---|---|---|---|---|---|---|---|---|
| Q96SN8(CK5P2);Q5VU 43(MYOME) | ND | ND | ND | ND | D | D | A | B |
| P04430(KV122) | C | C | C | C | D | D | B | C |
| Q9Y646(CBPQ) | C | C | C | C | C | C | B | C |
| Q9Y6U3(ADSV) | ND | ND | ND | ND | C | C | B | C |
| Q9Y624(JAM1) | ND | ND | ND | ND | C | C | B | C |
| P01619(KV301) | C | C | C | C | D | D | B | C |
| P48551(INAR2) | ND | ND | ND | ND | B | C | B | C |
| Q9HAT2(SIAE) | C | C | B | B | A | B | C | B |
| P54802(ANAG) | C | C | C | C | B | C | C | C |
| P17900(SAP3) | C | C | C | C | B | C | C | C |
| P81172(HEPC) | C | C | A | B | C | C | C | C |
| P04217(A1BG) | ND | ND | ND | ND | B | C | C | C |
| Q6UXB8(PI16) | ND | ND | ND | ND | B | C | c | C |
| P15289(ARSA) | B | B | C | C | A | B | C | C |
| P56537(IF6) | D | D | D | D | E | D | D | D |
| Q12794(HYAL1) | C | C | c | c | D | D | D | E |
| O75351(VPS4B) | D | D | D | D | D | D | D | E |
| Q09328(MGT5A) | ND | ND | ND | ND | D | D | D | E |
| P09871(C1S) | ND | ND | ND | ND | D | D | D | F |
| P08670(VIME);P17661( DESM);Q16352(AINX); P07197(NFM) | C | C | A | B | ND | ND | ND | ND |
| P01702(LV104);P06316 (LV107) | C | C | B | C | ND | ND | ND | ND |
| P10643(CO7) | F | F | E | E | ND | ND | ND | ND |

One skilled in the art readily appreciates that the present invention is well adapted to carry out the objects and obtain the ends and advantages mentioned, as well as those inherent therein.

The examples provided herein are representative of preferred embodiments, are exemplary, and are not intended as limitations on the scope of the invention. Modifications therein and other uses will occur to those skilled in the art.

All patents and publications mentioned in the specification are indicative of the levels of those of ordinary skill in the art to which the invention pertains.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof.

Other embodiments are set forth within the following claims.

## Claims

1. A method of diagnosing appendicitis in a subject, comprising:
performing an assay configured to detect Pro-Hepcidin on a body fluid sample obtained from the subject to provide an assay result; and
correlating the assay result to the occurrence or nonoccurrence of appendicitis in the subject by comparing the assay result to a threshold selected in a population study, wherein the threshold separates the population into a first subpopulation above the threshold which is at an increased predisposition for the occurrence of appendicitis, and a second subpopulation below the threshold which is at an increased predisposition for the nonoccurrence of appendicitis relative to the first subpopulation.

2. A method according to claim 1, wherein the performing step comprises introducing the body fluid sample obtained from the subject into an assay instrument which (i) contacts the body fluid sample with one or more binding reagents corresponding to Pro-Hepcidin, wherein the Pro-Hepcidin binds to the one or more binding reagents in an amount related to its concentration in the body fluid sample, (ii) generates an assay result indicative of binding of Pro-Hepcidin to the one or more binding reagents; and (iii) displays the assay result as a quantitative result in a human-readable form.

3. A method according to claim 1 or 2, wherein the subject is being evaluated for abdominal pain.

4. A method according to claim 2 or 3, wherein the assay instrument comprises a processing system configured to perform the correlating step and output the assay result or a value derived therefrom in human readable form.

5. A method according to one of claims 1-4, wherein the method provides a sensitivity or specificity of at least 0.7 for the identification of appendicitis when compared to normal subjects, or
wherein the method provides a sensitivity or specificity of at least 0.7 for the identification of appendicitis when compared to subjects exhibiting symptoms that mimic appendicitis symptoms.

6. A method according to one of claims 1-5, wherein the sample is selected from the group consisting of urine, blood, serum, and plasma.

7. A method for evaluating Pro-Hepcidin levels in a body fluid sample, comprising:
performing an analyte binding assay configured to detect Pro-Hepcidin in a body fluid sample obtained from a subject selected for evaluation based on a determination that the subject is experiencing symptoms indicative of possible acute appendicitis, wherein the body fluid sample is introduced into an assay instrument which (i) contacts the body fluid sample with one or more binding reagents, wherein Pro-Hepcidin binds to the one or more binding reagents in an amount related to its concentration in the body fluid sample, (ii) generates an assay result indicative of binding of Pro-Hepcidin to the one or more binding reagents, and (iii) displays the assay result as a quantitative result in a human-readable form.

8. A method according to claim 7, wherein the assay result is displayed as a concentration of Pro-Hepcidin,
wherein the assay instrument further compares the concentration of Pro-Hepcidin to a corresponding threshold level for Pro-Hepcidin, and displays an indication of whether Pro-Hepcidin does or does not exceed its corresponding threshold in a human-readable form.

9. A method for evaluating Pro-Hepcidin levels in a body fluid sample comprising:
performing an analyte binding assay configured to detect Pro-Hepcidin in a body fluid sample obtained from a subject selected for evaluation based on a determination that the subject has been diagnosed with acute appendicitis, wherein the body fluid sample is introduced into an assay instrument which (i) contacts the body fluid sample with one or more binding reagents, wherein Pro-Hepcidin binds to the one or more binding reagents in an amount related to its concentration in the body fluid sample, (ii) generates an assay result indicative of binding of Pro-Hepcidin to the one or more binding reagents, and (iii) displays the assay result as a quantitative result in a human-readable form.

10. A method according to one of claims 7-9, wherein the assay is an immunoassay performed by (i) introducing the body fluid sample into an assay device comprising an antibody which binds to Pro-Hepcidin, and (ii) generating an assay result indicative of binding of Pro-Hepcidin to the antibody.

11. A method according to one of claims 7-10, wherein the assay result indicates the body fluid sample has an elevated level of Pro-Hepcidin as compared to a predetermined threshold level.

12. Use of one or more reagents which specifically bind Pro-Hepcidin for the diagnosis of appendicitis.

13. Use of the biomarker Pro-Hepcidin for the diagnosis of appendicitis.

## Patentansprüche

1. Verfahren zur Diagnose von Appendizitis bei einem Subjekt, umfassend:
Durchführen eines Assays, der konfiguriert ist, um Pro-Hepcidin in einer von dem Subjekt erhaltenen Körperfluidprobe nachzuweisen, um ein Assay-Ergebnis bereitzustellen; und
Korrelieren des Assay-Ergebnisses mit dem Auftreten oder Nicht-Auftreten von Appendizitis in dem Subjekt durch Vergleichen des Assay-Ergebnisses mit einem in einer Populationsstudie ausgewählten Schwellenwert, wobei der Schwellenwert die Population in eine erste Teilpopulation oberhalb des Schwellenwerts, die eine erhöhte Prädisposition für das Auftreten von Appendizitis aufweist, und eine zweite Teilpopulation unterhalb des Schwellenwerts, die eine erhöhte Prädisposition für das Nicht-Auftreten von Appendizitis relativ zu der ersten Teilpopulation aufweist, trennt.

2. Verfahren nach Anspruch 1, wobei der Durchführungsschritt das Einbringen der von dem Subjekt erhaltenen Körperfluidprobe in ein Assay-Instrument umfasst, das (i) die Körperfluidprobe mit einem oder mehreren Pro-Hepcidin entsprechenden Bindungsreagenzien in Kontakt bringt, wobei das Pro-Hepcidin an das eine oder die mehreren Bindungsreagenzien in einer Menge bindet, die sich auf seine Konzentration in der Körperfluidprobe bezieht, (ii) ein Assay-Ergebnis erzeugt, das die Bindung von Pro-Hepcidin an das eine oder die mehreren Bindungsreagenzien anzeigt; und (iii) das Assay-Ergebnis als quantitatives Ergebnis in einer menschlich lesbaren Form anzeigt.

3. Verfahren nach Anspruch 1 oder 2, wobei das Subjekt auf Bauchschmerzen untersucht wird.

4. Verfahren nach Anspruch 2 oder 3, wobei das Assay-Instrument ein Verarbeitungssystem umfasst, das so konfiguriert ist, dass es den Korrelationsschritt durchführt und das Assay-Ergebnis oder einen davon abgeleiteten Wert in menschlich lesbarer Form ausgibt.

5. Verfahren nach einem der Ansprüche 1-4, wobei das Verfahren eine Sensitivität oder Spezifität von mindestens 0,7 für die Identifizierung von Appendizitis im Vergleich zu normalen Subjekten bereitstellt, oder
wobei das Verfahren eine Sensitivität oder Spezifität von mindestens 0,7 für die Identifizierung von Appendizitis im Vergleich zu Subjekten mit Symptomen, die Appendizitis-Symptome imitieren, bereitstellt.

6. Verfahren nach einem der Ansprüche 1-5, wobei die Probe aus der Gruppe ausgewählt ist bestehend aus Urin, Blut, Serum und Plasma.

7. Verfahren zur Bewertung des Niveaus von Pro-Hepcidin in einer Probe eines Körperfluids, umfassend:
Durchführen eines Analyt-Bindungsassays, der konfiguriert ist, um Pro-Hepcidin in einer Körperfluidprobe nachzuweisen, die von einem Subjekt erhalten wurde, das für eine Bewertung auf Basis einer Bestimmung ausgewählt wurde, dass das Subjekt Symptome erfährt, die auf eine mögliche akute Appendizitis hinweisen, wobei die Körperfluidprobe in ein Assay-Instrument eingeführt wird, das (i) die Körperfluidprobe mit einem oder mehreren Bindungsreagenzien in Kontakt bringt, wobei Pro-Hepcidin an das eine oder an mehrere Bindungsreagenzien in einer Menge bindet, die mit seiner Konzentration in der Körperfluidprobe in Beziehung steht, (ii) ein Assay-Ergebnis erzeugt, das die Bindung von Pro-Hepcidin an das eine oder an mehrere Bindungsreagenzien anzeigt, und (iii) das Assay-Ergebnis als quantitatives Ergebnis in einer menschlich lesbaren Form anzeigt.

8. Verfahren nach Anspruch 7, wobei das Assay-Ergebnis als eine Konzentration von Pro-Hepcidin angezeigt wird,
wobei das Assay-Instrument ferner die Konzentration von Pro-Hepcidin mit einem entsprechenden Schwellenniveau für Pro-Hepcidin vergleicht und einen Hinweis darauf, ob Pro-Hepcidin sein entsprechendes Schwellenniveau überschreitet oder nicht, in einer menschlich lesbaren Form anzeigt.

9. Verfahren zur Bewertung des Niveaus von Pro-Hepcidin in einer Probe eines Körperfluids, umfassend:
Durchführen eines Analyt-Bindungsassays, der konfiguriert ist, um Pro-Hepcidin in einer Körperfluidprobe nachzuweisen, die von einem Subjekt erhalten wurde, das auf Basis von einer Bestimmung, dass bei dem Subjekt eine akute Appendizitis diagnostiziert wurde, zur Bewertung ausgewählt wurde, wobei die Körperfluidprobe in ein Assay-Instrument eingeführt wird, das (i) die Körperfluidprobe mit einem oder mehreren Bindungsreagenzien in Kontakt bringt, wobei Pro-Hepcidin an das eine oder mehrere Bindungsreagenzien in einer Menge bindet, die mit seiner Konzentration in der Körperfluidprobe in Beziehung steht, (ii) ein Assay-Ergebnis erzeugt, das die Bindung von Pro-Hepcidin an das eine oder mehrere Bindungsreagenzien anzeigt, und (iii) das Assay-Ergebnis als quantitatives Ergebnis in einer menschlich lesbaren Form anzeigt.

10. Verfahren nach einem der Ansprüche 7 bis 9, wobei der Assay ein Immunoassay ist, der durchgeführt wird durch (i) Einbringen der Probe des Körperfluids in eine Assay-Vorrichtung, die einen Antikörper umfasst, der an Pro-Hepcidin bindet, und (ii) Erzeugen eines Assay-Ergebnisses, das die Bindung von Pro-Hepcidin an den Antikörper anzeigt.

11. Verfahren nach einem der Ansprüche 7-10, wobei das Assay-Ergebnis angibt, dass die Fluid-Probe ein erhöhtes Niveau von Pro-Hepcidin im Vergleich zu einem vorbestimmten Schwellenniveau aufweist.

12. Verwendung von einem oder mehreren Reagenzien, die spezifisch Pro-Hepcidin binden, für die Diagnose von Appendizitis.

13. Verwendung des Biomarkers Pro-Hepcidin für die Diagnose von Appendizitis.

## Revendications

1. Procédé pour diagnostiquer l'appendicite chez un sujet, comprenant :
la réalisation d'un dosage conçu pour détecter la Pro-Hepcidine dans un échantillon de fluide corporel obtenu du sujet pour fournir un résultat de dosage ; et
la corrélation du résultat de dosage avec l'occurrence ou la non-occurrence de l'appendicite chez le sujet par comparaison du résultat de dosage avec un seuil choisi dans une étude de population, le seuil séparant la population en une première sous-population au-dessus du seuil qui a une prédisposition accrue pour l'occurrence d'une appendicite, et une deuxième sous-population en dessous du seuil qui a une prédisposition accrue pour la non-occurrence d'une appendicite par rapport à la première sous-population.

2. Procédé selon la revendication 1, l'étape de réalisation comprenant l'introduction de l'échantillon de fluide corporel obtenu du sujet dans un instrument de dosage qui (i) met en contact l'échantillon de fluide corporel avec un ou plusieurs réactifs de liaison correspondant à la Pro-Hepcidine, la Pro-Hepcidine se liant au réactif ou aux réactifs de liaison en une quantité liée à sa concentration dans l'échantillon de fluide corporel, (ii) génère un résultat de dosage indicatif de la liaison de la Pro-Hepcidine au réactif ou aux réactifs de liaison ; et (iii) présente le résultat de dosage en tant que résultat quantitatif sous une forme lisible par un être humain.

3. Procédé selon la revendication 1 ou 2, le sujet étant évalué pour une douleur abdominale.

4. Procédé selon la revendication 2 ou 3, l'instrument de dosage comprenant un système de traitement configuré pour réaliser l'étape de corrélation et produire le résultat de dosage ou une valeur issue de celui-ci sous une forme lisible par un être humain.

5. Procédé selon l'une quelconque des revendications 1 à 4, le procédé fournissant une sensibilité ou spécificité d'au moins 0,7 pour l'identification d'une appendicite par comparaison à des sujets normaux, ou
le procédé fournissant une sensibilité ou spécificité d'au moins 0,7 pour l'identification d'une appendicite par comparaison à des sujets présentant des symptômes qui miment des symptômes d'appendicite.

6. Procédé selon l'une quelconque des revendications 1 à 5, l'échantillon étant choisi dans le groupe constitué par l'urine, le sang, le sérum et le plasma.

7. Procédé pour l'évaluation de taux de Pro-Hepcidine dans un échantillon de fluide corporel, comprenant
la réalisation d'un dosage de liaison d'un analyte conçu pour détecter la Pro-Hepcidine dans un échantillon de fluide corporel obtenu d'un sujet choisi pour une évaluation sur la base d'une détermination du fait que le sujet éprouve des symptômes indicatifs d'une possible appendicite aiguë, l'échantillon de fluide corporel étant introduit dans un instrument de dosage qui (i) met en contact l'échantillon de fluide corporel avec un ou plusieurs réactifs de liaison, la Pro-Hepcidine se liant au réactif ou aux réactifs de liaison en une quantité liée à sa concentration dans l'échantillon de fluide corporel, (ii) génère un résultat de dosage indicatif de la liaison de la Pro-Hepcidine au réactif ou aux réactifs de liaison ; et (iii) présente le résultat de dosage en tant que résultat quantitatif sous une forme lisible par un être humain.

8. Procédé selon la revendication 7, le résultat de dosage étant présenté comme une concentration de Pro-Hepcidine,
l'instrument de dosage comparant en outre la concentration de Pro-Hepcidine par rapport à un taux seuil correspondant pour la Pro-Hepcidine, et présentant une indication de si la Pro-Hepcidine dépasse ou ne dépasse pas son seuil correspondant sous une forme lisible par un être humain.

9. Procédé pour l'évaluation de taux de Pro-Hepcidine dans un échantillon de fluide corporel comprenant :
la réalisation d'un dosage de liaison d'un analyte conçu pour détecter la Pro-Hepcidine dans un échantillon de fluide corporel obtenu d'un sujet choisi pour une évaluation sur la base d'une détermination du fait que l'on a diagnostiqué une appendicite aiguë chez le sujet, l'échantillon de fluide corporel étant introduit dans un instrument de dosage qui (i) met en contact l'échantillon de fluide corporel avec un ou plusieurs réactifs de liaison, la Pro-Hepcidine se liant au réactif ou aux réactifs de liaison en une quantité liée à sa concentration dans l'échantillon de fluide corporel, (ii) génère un résultat de dosage indicatif de la liaison de la Pro-Hepcidine au réactif ou aux réactifs de liaison ; et (iii) présente le résultat de dosage en tant que résultat quantitatif sous une forme lisible par un être humain.

10. Procédé selon l'une des revendications 7 à 9, le dosage étant un dosage immunologique réalisé par
(i) introduction de l'échantillon de fluide corporel dans un dispositif de dosage comprenant un anticorps qui se lie à la Pro-Hepcidine, et
(ii) génération d'un résultat de dosage indicatif de la liaison de la Pro-Hepcidine à l'anticorps.

11. Procédé selon l'une des revendications 7 à 10, le résultat de dosage indiquant que l'échantillon de fluide corporel présente un taux élevé de Pro-Hepcidine par comparaison avec un taux seuil prédéterminé.

12. Utilisation d'un ou de plusieurs réactifs qui se lient spécifiquement à la Pro-Hepcidine pour le diagnostic d'une appendicite.

13. Utilisation du biomarqueur Pro-Hepcidine pour le diagnostic d'une appendicite.
